# EUROPÄISCHE PATENTSCHRIFT

(11) **EP 3 929 938 B1**
(45) Veröffentlichungstag und Bekanntmachung des Hinweises auf die Patenterteilung: **06.11.2024**
(21) Anmeldenummer: 21181537.8
(22) Anmeldetag: 24.06.2021
(51) Int. Cl.: G16H 40/20, G16H 10/40, G16H 50/80, G06Q 50/00, G06Q 10/06

(54) **SYSTEM, VERFAHREN UND EINRICHTUNG ZUM FAHRZEUGGESTÜTZTEN, MOBILEN ERFASSEN VON GESUNDHEITSDATEN**
SYSTEM, METHOD AND DEVICE FOR VEHICLE-BASED, MOBILE ACQUISITION OF HEALTH DATA
SYSTÈME, PROCÉDÉ ET DISPOSITIF D'ACQUISITION MOBILE DE DONNÉES DE SANTÉ ASSISTÉE PAR VÉHICULE

(30) Priorität: 25.06.2020 DE 102020116806; 13.11.2020 DE 102020130033; 30.12.2020 DE 102020135148; 18.03.2021 DE 102021106682
(43) Veröffentlichungstag der Anmeldung: 29.12.2021
(73) Patentinhaber: LeeLoo Medical GmbH, 12435 Berlin (DE)
(72) Erfinder: Hertel, Sven, 13187 Berlin (DE); Schurer, Marcus, 83104 Tuntenhausen (DE); Treichel, Kai, 10999 Berlin (DE); Nietschke, Jonas, 10967 Berlin (DE)
(74) Vertreter: Meissner Bolte Partnerschaft mbB

(56) Entgegenhaltungen:
- DE-A1- 102018 222 144
- GB-A- 2 477 799
- US-A1- 2011 093 249
- US-A1- 2013 078 624

## Beschreibung

Die Erfindung betrifft ein Verfahren und eine Einrichtung zum fahrzeuggestützten, mobilen Erfassen von Gesundheitsdaten durch Beprobung oder Testung natürlicher Personen.

Die Einrichtung umfasst hierbei eine Vielzahl von mobilen Einheiten zur Durchführung einer lokalen Infektionsdiagnostik.

Aus der DE 10 2018 222 144 A1 ist ein Verfahren zur rechnerbasierten Vermittlung von mobilen Dienstleistungen, die in Servicemobilen erbracht werden, vorbekannt.

Die Buchung einer mobilen Dienstleistung geschieht über ein Buchungsportal. Ein Verwaltungsserver des Dienstleistungsbuchungsportals empfängt eine Anfrage zur Erbringung der mobilen Dienstleistung von einem Kunden. Der Verwaltungsserver stellt eine entsprechende Suchanfrage an eine Servicemobil-Datenbank, in der die verfügbaren Servicemobile verzeichnet sind.

Der Verwaltungsserver nimmt dabei eine Anpassung der Servicemobil-Parameter vor, und zwar auf der Basis der in einer Kundenstammdatenbank verzeichneten Kundenzusatzinformationen zu den jeweiligen Kunden. Der Verwaltungsserver stellt dem Kunden eine Auswahl von potentiell geeigneten Servicemobilen entsprechend der angepassten Servicemobil-Parameter zur Verfügung und reserviert das von dem Kunden ausgewählte Servicemobil.

Bei den Dienstleistungen, die während der Bewegung selbstfahrender Servicemobile erbracht werden können, handelt es sich zum Beispiel um ein mobiles Friseur- und Kosmetikstudio, einen Beratungs- oder Coachingbereich in einem Fahrzeug oder aber auch um das Anbieten von Speisen oder Getränken in Fahrzeugen.

Die DE 10 2019 126 370 A1 zeigt die Möglichkeit auf, dass ein Dienstleister, wie etwa ein Beförderungsverwaltungsdienst, die Beförderung für eine Reihe von Fahrgästen, einschließlich Fahrgästen mit besonderen Bedürfnissen, zwischen verschiedenen Standorten verwalten kann.

Einzelne Routen können mindestens zwei Segmente enthalten, die den gleichen oder unterschiedlichen Beförderungsmodi verwenden. Ein Kunde kann eine Beförderungsoption für die Fahrt auswählen, was dazu führt, dass Reservierungen für die verschiedenen Segmente vorgenommen werden. Auf Grundlage der Bedürfnisse eines Fahrgastes können die Beförderungsoptionen auf solche begrenzt werden, die Fahrzeuge verwenden, welche die Bedürfnisse des Fahrgastes berücksichtigen.

Die DE 10 2019 126 370 A1 offenbart die Option, über die ein Benutzer zusätzliche Informationen bezüglich des Gesundheitszustandes des Benutzers bereitstellen kann. Beispielsweise kann ein Benutzer entscheiden, Notfall-Kontaktinformationen, die Kontaktinformationen eines Arztes oder Informationen bezüglich eines Gesundheitsproblems bereitzustellen. Beispielsweise kann ein Fahrgast, wenn ihm bekannt ist, dass ein Risiko von epileptischen Anfällen besteht, dieses Problem beschreiben und Anweisungen zur Verwendung eines Epinephrin-Autoinjektors bereitstellen, den er bei sich trägt. Wenn dieser Fahrgast einen Anfall hat, wäre der Fahrer oder anderes Personal in der Lage, relevante Anweisungen zur Bereitstellung einer Behandlung über die Anwendung abzufragen oder entsprechende Notfallkontakte oder medizinisches Fachpersonal zu kontaktieren.

Bei dem Verfahren und dem System zum Auswählen von Navigationsrouten und Bereitstellen von Werbung auf der Route gemäß DE 10 2014 203 724 A1 kann eine Anzahl von der einen oder den mehreren Navigationsrouten zugeordneten Fahrmanövern, um das Ziel zu erreichen, im Vorfeld bestimmt werden. Das Verfahren kann außerdem einen Arbeitslastwert für das Fahrmanöver entsprechend der einen oder der mehreren Navigationsrouten bestimmen und auf der Basis des Arbeitslastwertes die Navigationsroute auswählen. Bei einer Ausführungsform kann das Navigationssystem Daten über eine geeignete Schnittstelle übernehmen und in ein Speichermedium ablegen, bis diese Daten nicht mehr benötigt werden. Außerdem besteht die Möglichkeit einer Kommunikation mit vielfältigen Hilfseinrichtungen. Solche Hilfseinrichtungen können persönliche Medien-Player, aber auch drahtlose Gesundheitseinrichtungen, tragbare Computer oder dergleichen umfassen.

Bekannt ist auch eine mobile Geschäftsstelle für ein Finanzinstitut gemäß EP 1 728 679 B1. Diese mobile Geschäftsstelle kann ergänzend oder alternativ zu bislang üblichen ortsgebundenen Geschäftsstellen zum Einsatz kommen.

Bei einer konkreten Ausführungsform weist die mobile Geschäftsstelle einen Auflieger auf, der dazu vorgesehen ist, von einer Zugmaschine bewegt zu werden. Der Innenraum des Aufliegers ist hier über einen Kundenzugang zugänglich. Weiterhin kann der Innenraum des Aufliegers über einen separaten Notausgang verlassen werden. Der Innenraum des Aufliegers ist in zumindest drei voneinander abgeteilte Zonen unterteilt, nämlich in einen Kassen- oder Schalterraum, eine Sanitärzone und eine Kundenberatungszone mit Sitzgelegenheiten. Weiterhin umfasst die mobile Geschäftsstelle alle Einrichtungen, die für einen ordnungsgemäßen Betrieb einer solchen Geschäftsstelle eines Finanzinstitutes erforderlich sind.

Insofern ist die Geschäftsstelle mit Kommunikationsmitteln zum Herstellen einer bidirektionalen satellitengestützten Datenkommunikation zu einem entfernten Rechner ausgerüstet.

Grundsätzlich besteht in pandemischen Situationen, die sich territorial sehr schnell und mit teilweise nicht vorhersehbaren Folgen ausbreiten, die Notwendigkeit direkt und schnell, auch in strukturschwachen Regionen oder Hot Spots in ausreichender Anzahl und Menge Testmöglichkeiten zu schaffen. Diese gewünschten mobilen Testzentren sollen insbesondere auch ältere oder erkrankte Personen, und insbesondere Personen, die sogenannte Risikopatienten sind, erreichen. Dabei gilt es, eine schnelle Untersuchung von Patienten durchzuführen und für einen optimalen Datenaustausch mit Gesundheitszentren, Krankenhäusern und sonstigen involvierten Behörden zu bewerkstelligen.

Bei einem akuten Verdacht und Gefahr der Infizierung von Menschen im Umfeld des Verdachtsfalles soll schnell und gezielt reagiert und eine Ermittlung der Infektionsraten erfolgen können, damit einer Verbreitung zügig entgegengewirkt werden kann.

Aus dem Vorgenannten ist es Aufgabe der Erfindung, ein weiterentwickeltes Verfahren und eine Einrichtung zum fahrzeuggestützten, mobilen Erfassen von Gesundheitsdaten anzugeben. Dabei sollen die Forderungen für eine Diagnostik in einem Fahrzeug im Sinne eines direkten Erregernachweises aus einem Abstrich oder dem Sputum erfüllt werden. Die Aufbereitung von Untersuchungsproben im Fahrzeug und der Gesundheitsschutz der dort tätigen Personen müssen den Anforderungen des Ausschusses für biologische Arbeitsstoffe entsprechen. Das System soll neben dem mobilen Aufsuchen und einer mobilen Diagnostik von Patienten in der Fläche auch eine Vernetzung mit und Entlastung von regionalen Gesundheitsämtern realisieren.

Die Labordiagnostik und die Speicherung von Patientendaten soll im Fahrzeug erfolgen, wobei eine Vernetzung der Fahrzeuge für einen Einsatz an sogenannten Hot Spots und eine Vernetzung mit Krankenhausinformationssystemen nebst Datenweitergabe an Gesundheitsämter ebenfalls im Blickfeld der Erfindung ist. Die Lösung der Aufgabe der Erfindung erfolgt durch die jeweilige Lehre der unabhängigen Ansprüche, wobei die Unteransprüche mindestens zweckmäßige Ausgestaltungen und Weiterbildungen umfassen.

Erfindungsgemäß erfolgt zunächst das Senden einer Anforderungsmitteilung an eine Flottenmanagementzentrale. Diese Anforderungsmitteilung kann von einem Arzt, einer Behörde aber auch von natürlichen Personen abgesetzt werden.

Danach wird ein Beurteilen und Lokalisieren der Anforderung durch die Flottenmanagementzentrale vorgenommen.

Ziel ist es hier, festzustellen, aus welchem Territorium die Anfrage kommt, ob es bereits Risikobetrachtungen zu dem Territorium gibt, ob womöglich bereits mobile Einheiten in der Nähe des ermittelten Territoriums befindlich sind und ob womöglich Schwierigkeiten bestehen, das entsprechende Territorium in einer abschätzbaren Zeit mit der mobilen Einheit, das heißt einem Fahrzeug zu erreichen.

Daraus folgt dann ein Überprüfen der Bereitschaft und des Standortes von mobilen Einheiten, die dann die eigentliche Beprobung und Testung mit entsprechend ausgebildetem Personal oder aber auch automatisiert vornehmen können.

In Folge wird eine oder je nach Umfang der Anforderung auch mehrere der mobilen Einheiten ausgewählt, und es wird ein entsprechender Fahrauftrag an die ausgewählte Einheit abgesetzt und zwar einschließlich einer Routenvorgabe.

Bevorzugt über die Flottenmanagementzentrale wird dann eine Überwachung des Fahrweges der jeweiligen mobilen Einheit vorgenommen. Weiterhin erfolgt ein Senden von Informationen an die Anforderungsmitteilungsstelle und/oder die jeweilige natürliche Person, die die Anforderung ausgelöst hat oder die zu testen ist.

Ergänzend kann eine Meldung der voraussichtlichen Ankunftszeit vorgenommen werden, um eine Wartezeit der Person bzw. ein unnötiges in Kontakt kommen mit weiteren Personen zu vermeiden.

Parallel erfolgt ein Überwachen möglicher weiterer eingegangener Anforderungsmitteilungen und das Erstellen eines Vorlaufzeitplanes für das Flottenmanagement.

Letztendlich wird ein pick-up-Ort bestimmt, um an diesem Ort eine kurzzeitige probenrelevante Aufnahme der betreffenden natürlichen Person in die mobile Einheit vorzunehmen.

In der mobilen Einheit wird dann die Durchführung der Beprobung oder des Testes vorgenommen, und es erfolgt ein Erfassen sowie Überprüfen personenrelevanter Daten nebst Überführen dieser Daten in ein zentrales Register oder die Cloud.

Die Auswertung der Beprobung und/oder des Testes kann bereits in der mobilen Einheit während der Fahrt gemäß Routenvorgabe erfolgen.

Die Testergebnisse werden dann in das zentrale Register oder die Cloud weitergeleitet.

Wenn eine Anpassung der Routenvorgabe aufgrund aktueller Vorgaben durch die Flottenmanagementzentrale erforderlich ist, wird dies an die jeweilige mobile Einheit weitergegeben.

Im Laufe der Einsatzzeit der mobilen Einheit können dann weitere Zyklen durchlaufen werden, wie oben erläutert und entsprechende Beprobungen und Tests durch Anfahren gemäß Routenvorgabe und Anlaufen der pick-up-Orte erfolgen.

Im Anschluss an einen Einsatzzyklus erfolgt dann ein technischer und hygienischer Support für die jeweilige mobile Einheit zur Herstellung der Wiedereinsatzfähigkeit für einen nächsten Einsatzzyklus.

Das erfindungsgemäße System umfasst also die Gesamtheit von Schritten und Maßnahmen zur Durchführung einer lokalen Infektionsdiagnostik in einem Fahrzeug mit modularem Aufbau zum Abstreichen und Testen sowie weiteren medizinisch diagnostischen Arbeitsschritten.

Als wesentliches Merkmal stellt sich das Testen direkt im Fahrzeug dar. Dieses Testen umfasst die notwendigen Abstriche, den eigentlichen Testvorgang und die Diagnostik. All diese Maßnahme geschehen vor Ort im Fahrzeug. Die entsprechenden Testergebnisse liegen dann in der mobilen Einheit, das heißt dem Fahrzeug, vor.

Beispielhaft besteht die Möglichkeit, sogenannte PCR-Schnelltestgeräte einzusetzen. Hier stehen die Testergebnisse in überschaubar kurzer Zeit zur Verfügung.

Die erfindungsgemäße Einrichtung umfasst ein Automotive-fähiges, schwingungsarmes Aufnahmesystem zum mobilen Betrieb von im Regelfall hochempfindlichen medizinischen Testgeräten. Das Aufnahmesystem ist modular bestückbar und erfüllt die Anforderung an hygiene- und seuchenschutzgerechte Standards im laufenden Einsatz.

Die Datenerfassung erfolgt durchgängig und umfasst ein Datenmanagement aller dem diagnostischen Zweck dienender Daten für den intersektoralen und multidisziplinären Zweck. Entsprechende Daten können Online an einen zu befundenden Arzt übertragen werden.

Die Ausgestaltung des Fahrzeuges als mobile Einheit umfasst die Sicherstellung der Energieversorgung der Testgeräte, das Einhalten der Hygienestandards und das Vorsehen der notwendigen IT-Infrastruktur nebst Bedieneinrichtungen.

Weiterhin ist im Fahrzeug ein Aufbau im Sinne mehrerer räumlicher Sektoren realisiert einschließlich einer Schleusenfunktion, um zu verhindern, dass Probanden bzw. Patienten mit dem Personal, insbesondere dem medizinischen Personal, aber auch mit dem Fahrer in Kontakt kommen. Insofern ist das Fahrzeug mit all seinen Komponenten autark ausgestaltet, verfügt über die notwendigen Reinigungs- und Desinfektionsmittel einschließlich einer Energieversorgung für den Notfall.

Alle Einbauten und Modifikationen des jeweiligen Fahrzeuges sind so umgesetzt, dass eine Reinigung und Desinfektion derjenigen Bestandteile, mit denen das Personal bzw. die Probanden in Kontakt kommen, in kurzer Zeit und in ausreichendem Maß erfolgen kann.

Insbesondere besteht die Möglichkeit eines Aktivierens von Desinfektionsgeräten auf der Basis energiereicher UV-Strahlung. Dabei ist sichergestellt, dass eine UV-Strahlungsdesinfektion nur dann erfolgen kann, wenn sich keine Personen im Strahlungsbereich, insbesondere im oder am Fahrzeug befinden.

Um eine Datenerfassung und Datenübertragung auch in weniger dicht besiedelten Gebieten mit nicht ausreichender funktechnischer Versorgung sicherzustellen, besteht die ausgestaltende Möglichkeit, im Fahrzeug Mittel zur Funk-Reichweitenvergrößerung, zum Beispiel Repeater, zu installieren. Ergänzend zu klassischen GSM- oder UMTS-Funkzellenverbindungen weist die mobile Einheit eine Anlage zur Satellitenkommunikation auf.

Durch eine im Fahrzeug vorgesehene Möglichkeit des Aufbaus eines WLAN-Netzes bzw. von Bluetooth-Verbindungen können Testergebnisse und Patientendaten unter Rückgriff auf notwendige Applikationssoftware unmittelbar auf ein mobiles Endgerät des Patienten oder Probanden übertragen oder von diesem mobilen Endgerät übernommen werden. Hierdurch ist eine wesentlich funktionale Erweiterung einer bereits bestehenden sogenannten Corona-App möglich.

Wenn gemäß einer vorgegebenen Route über ein integriertes Navigationssystem von einer Leitstelle aktuelle ergänzende Informationen zur Mobileinheit übertragen werden, kann kurzfristig eine Anpassung der Fahrtroute mit dem Ziel der Optimierung des Einsatzes der Fahrzeuge, einer Verkürzung der Fahrzeit insgesamt und einer schnelleren Kontaktaufnahme zu dem oder den Patienten, die getestet werden sollen, erfolgen.

Um auch in den klimatisch ungünstigen, kalten Jahreszeiten eine Testung einer Gruppe von Patienten sicherzustellen, können an oder im Fahrzeug Mittel zum Aufstellen eines, in einzelne Bereiche unterteilten Vorzeltes oder dergleichen vorgesehen sein, so dass wartende Patienten nicht unnötig Witterungsunbilden ausgesetzt sind, gleichzeitig jedoch der notwendige hygienische Abstand zwischen den Patienten innerhalb einer diesbezüglichen Wartezone gewährleistet werden.

Der notwendige Abstrich für den jeweiligen Test kann automatisiert ohne menschlichen Eingriff, das heißt durch Einsatz eines mechanischen Roboters, erfolgen, der dann den Abstrich durch eine Schleuse zum Testgerät befördert, ohne dass es hierbei weiterer Handlungen einer Laborperson bedarf.

Hieraus ergibt sich der Vorteil, dass die mit der Testung befassten Personen befreit sind von einer ansonsten bestehenden Notwendigkeit des Tragens einer Ganzkörper-persönlichen Schutzausrüstung. Eine Ermüdung und möglichen gesundheitlichen Schäden dieser Personen ist hierdurch vorgebeugt.

Diesbezüglich kann im Fahrzeug ein Sektor oder Abschnitt ausgebildet werden, der vom Patienten betreten wird. Je nach bestimmter Körpergröße wird dann eine Fixiervorrichtung eingestellt, auf der beispielsweise das Kinn des Patienten ruht und der Kopf einer leichten Fixierung unterliegt.

Nach einem optisch oder akustisch erfolgenden Signal wird der Patient aufgefordert, den Mund zu öffnen. Ein Teststäbchen fährt dann aus einer Stäbchenaufnahmevorrichtung heraus und nimmt den entsprechenden Abstrich vor.

Das Teststäbchen fährt dann zurück und wird automatisch dem Analysegerät über eine Transportvorrichtung bewegt, so dass die Test- und Analyseprozedur eingeleitet werden kann. Nach Rückfahren der Fixiervorrichtung kann der Patient den betreffenden Sektor verlassen und sein Testergebnis abwarten.

Der Aufbau der mobilen Einrichtung ist so konzipiert, dass der Patient möglichst keine Gegenstände, wie zum Beispiel Türgriffe oder ähnliches berühren muss. Insofern ist eine Sensorik zum Öffnen des Zuganges zum Testsektor vorhanden.

Optoelektronische Mittel erkennen, ob und wann sich ein Patient im Testsektor befindet und ob er die richtige Position zum Abstrich eingenommen hat. Manuelle Handlungen des Patienten, wie zum Beispiel betätigen von Stellelementen oder Auslöseknöpfen bzw. Schalteinrichtungen können entfallen.

Ungeachtet dieser vorbeugenden Maßnahmen zur Verhinderung einer Schmierinfektion besteht selbstverständlich die Möglichkeit, im oder an der mobilen Einheit einen Spender zur Ausgabe von Desinfektionsmitteln vorzusehen.

Dabei kann es sich insbesondere um solche Spender zur Aufnahme von Desinfektionsmitteln handeln, die beim Auftragen auf die Hände dann einen Farbumschlag von farbig in neutral ergeben, wenn eine ausreichende Einwirkzeit vorliegt. Durch die zunächst gegebene Farbigkeit des Desinfektionsmittels kann auch überprüft werden, ob alle Oberflächen einer Hand, zum Beispiel Zwischenräume an den Fingern, ausreichend benetzt wurden.

Am oder in der mobilen Einheit können Displays für wartende Patienten vorgesehen sein, um beispielsweise eine Wartezeit bis zum Test oder aber auch eine Wartezeit bis zum Erhalt und zur Ausgabe der Testergebnisse darzustellen.

Wenn zum Beispiel eine erfolgreiche Bluetooth-Übertragung eines Testergebnisses zu einem mobilen Endgerät des entsprechenden Patienten erfolgte, kann dieser höflich aufgefordert werden, den Wartebereich und das Umfeld des Fahrzeuges zu verlassen.

In Weiterbildung der Erfindungslehre besteht die Möglichkeit, neben einer Schnelltestung die mobile Einrichtung auch für weitere notwendige Grunduntersuchungen oder aber auch zum Verabreichen von Impfungen zu nutzen. Auch bei derartigen Anwendungen ist durch das vorgestellte Prinzip der mobilen Einrichtung die Privatsphäre des jeweiligen Patienten gesichert.

Ein beispielhafter Aufbau der Einrichtung, das heißt der mobilen Einheit, geht davon aus, einen besten Schutz von Patient, Mitarbeiter und Fahrer zu gewährleisten.

Fahrerraum, Laborraum im hinteren Bereich und Patientenraum fahrzeugmittig sind entsprechend räumlich getrennt und in Sektoren eingeteilt.

Insofern sind also die Kabine des Fahrerraums vom Labor und vom Patientenraum separiert. Der Fahrerraum ist nur durch Fahrer- und Beifahrertür erreichbar. Der vom Heck zugängliche Laborraum weist den notwendigen Aufbau auf nebst beispielsweise Handwaschbecken, technisches und medizinisches Equipment, Kommunikation zur Flotten-Cloud und ähnliches.

Der Patientenraum ist durch eine Schiebetür betretbar. Die Sektoren Laborraum und Patientenraum gewährleisten einen Zutritt von Tageslicht. Die Fenster sind jedoch im Sinne der gewünschten Privatsphäre mit der Möglichkeit des Abdunkelns versehen. Eine Versorgung mit Frischluft kann beispielsweise über ein Hubdach realisiert werden. Bei einer Ausführung eines Testmobils zum behindertengerechten Einsatz ist entweder das gesamte Fahrzeug absenkbar oder es besteht die Möglichkeit, den Zugang zum Patientenraum abzusenken oder mit einem Liftzugang auszugestalten.

Die Erfindung soll nachstehend anhand eines Ausführungsbeispieles sowie unter Zuhilfenahme von Figuren näher erläutert werden.

Die Figuren zeigen einen beispielhaften Aufbau eines Fahrzeugs als mobile Testeinheit sowie verschiedene Darstellungen eines als Rack ausgebildeten Aufnahmesystems für das Test-Equipment.

Im Einzelnen zeigen:
- Fig. 1: eine perspektivische Ansicht auf das Grundgestell des Aufnahmesystems;
- Fig. 2a, b: Ansichten von Ober- und Unterschale zur Aufnahme eines Testgerätes, um dieses im Grundgestell zu fixieren;
- Fig. 3: eine Fahrzeuginnenansicht mit jeweils fahrzeuglängsseitig installierten, mit einer Verkleidung versehenen Racks zur Aufnahme von beispielhaft sechzehn Testgeräten; und
- Fig. 4: eine Teil-Innenansicht eines Fahrzeuges in Richtung Fahrzeugfront mit Rackausstattung und Labormobiliar.

Das Grundgestell zur Bildung des Racks zur Aufnahme für das Test-Equipment besteht aus einer Schweißkonstruktion, basierend auf Vierkant-Hohlprofilen.

Das Grundgestell umfasst einen Rahmen 1, welcher im Bereich von Standfüßen Anbindeplatten 2 zur Verbindung mit der betreffenden Fahrzeugbodenbaugruppe aufweist.

Darüber hinaus sind Winkelelemente 3 vorhanden, die dem Befestigen des Racks an der Karosserieseitenwand dienen.

Ein Träger 4 dient der Anbindung eines Bedienpaneels.

Zwei innerhalb des Rahmens 1 befestigte Träger 5 bilden eine Anzahl von n-Aufnahmen für die entsprechenden Testgeräte.

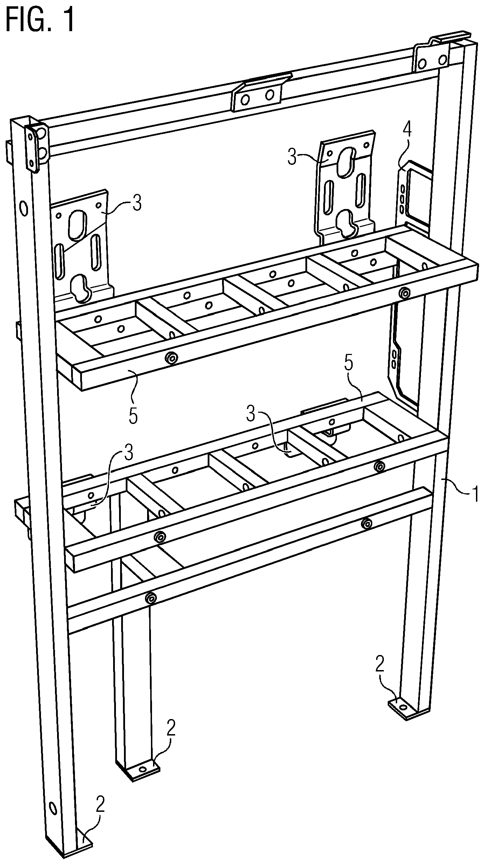

Im Beispiel gemäß der Figur 1 weist jeder Träger 5 vier Aufnahmen für vier Testgeräte auf, so dass insgesamt bei einem Rack acht Aufnahmemöglichkeiten für Testgeräte vorhanden sind.

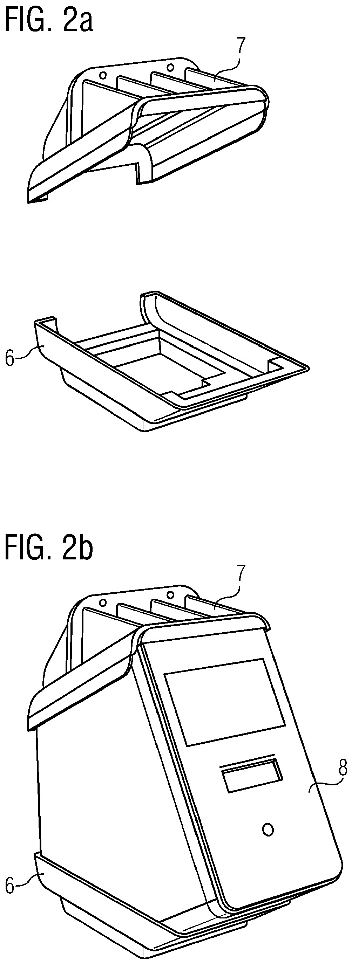

Die Figur 2a zeigt die Ausbildung einer Unterschale 6 sowie einer Oberschale 7.

Die Unterschale 6 ist maßlich auf die entsprechende Aufnahme im Träger 5 abgestimmt.

Innenseitig jeder Schale 6; 7 befindet sich ein schwingungsdämpfender Einsatz oder ein schwingungsdämpfendes Material.

Darüber hinaus kann das Material der Ober- oder Unterschale 6; 7 bereits schwingungsdämpfende Eigenschaften besitzen.

Die Innenoberflächen von Unterschale 6 und Oberschale 7 sind entsprechend der Kontur des Testgerätes 8 (siehe Figur 2b) ausgeführt und halten dieses sicher. Die Ausrichtung des Testgerätes 8 unter einem erkennbaren Winkel (siehe Figur 2b sowie die Figuren 3 und 4) sichern für den Bediener einen ergonomischen Zugang, um ermüdungsfrei Testkartuschen einführen und eine entsprechende Bedienung vornehmen zu können.

Die Oberschale 7 dient dazu, das Testgerät 8 in der gewünschten Position zu halten und ein Kippmoment zu eliminieren.

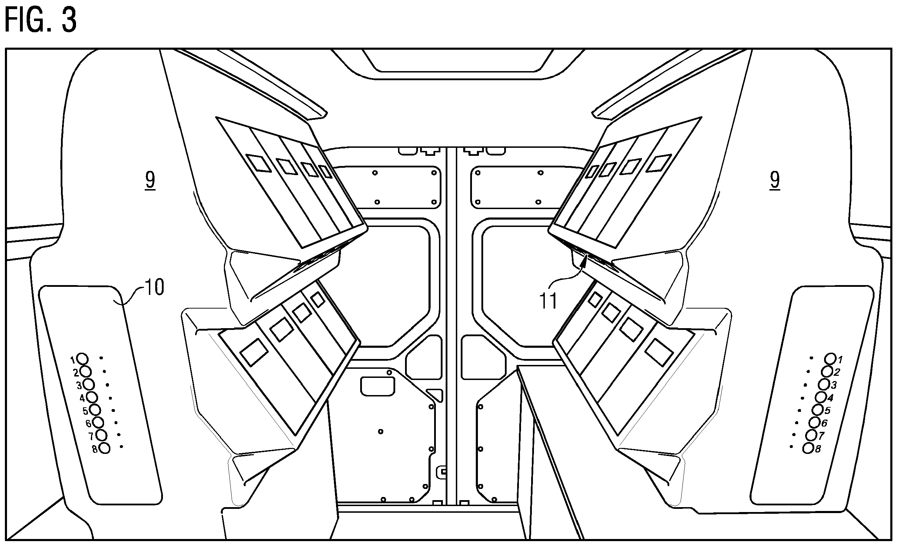

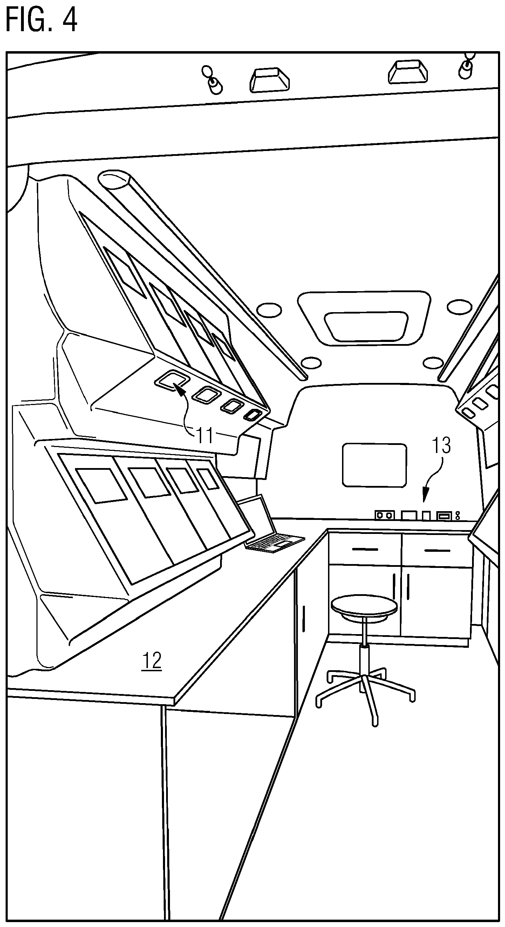

Die Figuren 3 und 4 zeigen jeweils Innenansichten bzw. Teil-Innenansichten eines Fahrzeuges, ausgerüstet mit dem vorgestellten Rack zur Aufnahme des notwendigen Test-Equipment, das heißt der Testgeräte 8 und weiterer Laborkomponenten.

Das Grundgestell mit den Testgeräten wird von einer, die Innenkontur des entsprechenden Fahrzeuges aufnehmenden Umhausung 9 verschlossen. Die Umhausung 9 besteht aus einem den hygienischen Anforderungen genügenden Material mit einer leicht zu reinigenden Oberfläche.

An einer Stirnseite der Umhausung 9 befindet sich ein Zugang zur Aufnahme des Bedienpaneels 10.

Bei einer erfindungsgemäßen Variante besteht die Verkleidung bzw. das Gehäuse 9 aus einem Laminat, welches mit einem antibakteriellen Lack beschichtet wurde. Weiterhin sind Frontpaneele vorhanden, die aus PMMA-Material gefertigt werden können, welches ebenso leicht zu reinigen ist.

Um eine Abdichtung zu den Testgeräten zu gewährleisten, besitzen die Paneele entsprechende Dicht- bzw. Dämmmaterialien, zum Beispiel aus EPDM.

Unterseitig vorgesehene Scanner 11 (siehe Figur 3) sind ebenso vom Gehäuse 9 verkleidet und besitzen eine Blende nebst Abdichtung. Hierdurch ist einerseits der Betrieb der Scanner möglich und andererseits eine schnelle Oberflächenreinigung möglich.

Unterhalb der Racks, welche die Testgeräte 8 und das Gehäuse 9 aufnehmen, verbleibt Raum für entsprechende Labormöbel 12.

Weitere technische Komponenten 13 können im Bereich hin zur Fahrzeugfront angeordnet werden.

## Patentansprüche

1. Verfahren zum und für einen fahrzeuggestützten, mobilen
Erfassungsvorgang von Gesundheitsdaten durch Beprobung oder Testung natürlicher Personen in pandemischen Situationen die sich territorial ausbreiten, wobei ein Senden einer Anforderungsmitteilung an eine Flottenmanagementzentrale und ein Bewerten und Lokalisieren der Anforderung durch die Flottenmanagementzentrale erfolgt,
**dadurch gekennzeichnet, dass**
festgestellt wird, aus welchem Territorium die Anforderungsmitteilung kommt, ob es bereits Risikobetrachtungen zu diesem Territorium gibt, ob bereits mobile Einheiten in der Nähe des Territoriums befindlich sind und ob es möglich ist, das entsprechende Territorium in einer abschätzbaren Zeit mit der mobilen Einheit, d. h. einem Fahrzeug zu erreichen, anschließendes Überprüfen der Bereitschaft und des Standortes von mobilen Einheiten zur Beauftragung einer Beprobung und Testung, Auswählen einer der mobilen Einheiten und Absetzen eines Fahrauftrages an die ausgewählte Einheit sowie Übermittlung einer Routenvorgabe an die mobile Einheit,
Überwachen des Fahrweges der jeweiligen mobilen Einheit durch die Flottenmanagementzentrale und Senden von Informationen an die jeweilige natürliche Person nebst Meldung einer voraussichtlichen Ankunftszeit zur Vermeidung von Wartezeiten und unnötiger Kontakte mit weiteren Personen, paralleles Überwachen möglicher weiterer eingegangener Anforderungsmitteilungen und Erstellen eines Vorlaufzeitplanes für das Flottenmanagement durch Anpassung der Fahrtroute zur Optimierung des Einsatzes der Fahrzeuge, Verkürzung der Fahrzeit und schnelle Kontaktaufnahme mit den zu testenden Personen,
Bestimmen eines pick-up-Ortes zur kurzzeitigen, probenrelevanten Aufnahme der betreffenden natürlichen Person in die mobile Einheit, Durchführung der Beprobung und/oder der notwendigen Tests und Erfassen sowie Überprüfen personenrelevanter Daten nebst Überführen dieser Daten in ein zentrales Register oder die Cloud,
Auswertung der Beprobung oder des Testes bereits in der mobilen Einheit während der Fahrt gemäß Routenvorgabe,
Weiterleiten der Beprobungs- oder Testergebnisse in das zentrale Register oder die Cloud,
Anpassung der Routenvorgabe anhand aktueller Vorgaben durch die Flottenmanagementzentrale,
im Laufe der Einsatzzeit der mobilen Einheit Durchlaufen weiterer Zyklen von Beprobungen und/oder Tests durch Anfahren gemäß Routenvorgabe und Anlaufen der pick-up-Orte sowie technischer und hygienischer Support für die mobile Einheit zur Herstellung der Wiedereinsatzfähigkeit für einen nächsten Einsatzzyklus mittels Reinigung und Desinfektion.

2. Verfahren nach Anspruch 1,
**gekennzeichnet durch**
den Einsatz einer Vielzahl von mobilen Einheiten zur Durchführung von Abstrichen für die Testung automatisiert ohne menschlichen Eingriff durch Einsatz eines mechanischen Roboters.

3. Einrichtung mit Mitteln konfiguriert zur Durchführung eines Verfahrens nach Anspruch 2
**gekennzeichnet durch**
ein Fahrzeug mit modularem Aufbau zum Aufnehmen von Abstrichen und Testen sowie zur temporären Aufnahme von Patienten und Laborpersonal, wobei ein Fahrerraum, ein Laborraum und ein Patientenraum räumlich getrennt in Sektoren ausgebildet sind.

4. Einrichtung nach Anspruch 3,
**gekennzeichnet durch**
ein schwingungsarmes Aufnahmesystem zur Integration von medizinischen Testgeräten für deren Einsatz im mobilen Betrieb.

5. Einrichtung nach Anspruch 3 oder 4,
**dadurch gekennzeichnet, dass**
die Einrichtung Mittel zur Übertragung von Testergebnissen und Patientendaten unmittelbar auf ein mobiles Endgerät des Patienten aufweist.

## Claims

1. A method to and for a vehicle-based, mobile acquisition process of health data by sampling or testing of natural persons in pandemic situations which spread territorially, wherein a transmitting of a request notification to a fleet management centre and an evaluating and locating of the request takes place through the fleet management centre,
**characterized in that**
it is established from which territory the request notification comes, whether risk considerations already exist regarding this territory, whether mobile units are already situated in the vicinity of the territory and whether it is possible to reach the corresponding territory with the mobile unit, i.e. a vehicle, in an estimable time,
subsequent checking of the readiness and of the location of mobile units for commissioning a sampling and testing,
selecting one of the mobile units and placing a travel order to the selected unit and delivering a route specification to the mobile unit,
monitoring the route of the respective mobile unit through the fleet management centre and transmitting information to the respective natural person together with notification of an expected arrival time, to avoid waiting times and unnecessary contacts with further persons,
parallel monitoring of possible further received request notifications and drawing up a lead time plan for the fleet management by adapting the travel route for optimization of the use of the vehicles, shortening of the travel time and rapid contact with the persons who are to be tested,
determining a pick-up location for the brief, sample-relevant admission of the respective natural person into the mobile unit,
carrying out the sampling and/or the necessary tests and recording and checking person-relevant data together with transferring these data into a central register or the cloud,
evaluation of the sampling or of the test already in the mobile unit during travel in accordance with route specification,
forwarding the sampling- or test results into the central register or the cloud,
adapting the route specification by means of current specifications through the fleet management centre,
running through further cycles of samplings and/or tests in the course of the operating time of the mobile unit by setting off in accordance with route specification and calling at the pick-up locations and technical and hygiene support for the mobile unit for restoring readiness for re-use for a next usage cycle by means of cleaning and disinfection.

2. The method according to Claim 1,
**characterized by**
the use of a plurality of mobile units for the carrying out of smears for testing in an automated manner without human intervention through the use of a mechanical robot.

3. A device with means configured for carrying out a method according to Claim 2,
**characterized by**
a vehicle with modular construction for the receiving of smears and tests and for the temporary admission of patients and laboratory personnel, wherein a driver's compartment, a laboratory compartment and a patient compartment are configured in sectors in a spatially separated manner.

4. The device according to Claim 3,
**characterized by**
a low-vibration receiving system for the integration of medical test apparatus for the use thereof in mobile operation.

5. The device according to Claim 3 or 4,
**characterized in that**
the device has means for the transmitting of test results and patient data directly to a mobile terminal of the patient.

## Revendications

1. Procédé en vue de et pour l'acquisition mobile de données de santé assistée par véhicule par échantillonnage ou des tests de personnes naturelles dans des situations pandémiques qui se répandent sur le territoire, dans lequel un envoi d'un message de demande à une centrale de gestion de flotte et une évaluation et une localisation de la demande par la centrale de gestion de flotte sont effectués, **caractérisé en ce qu'**il est constaté de quel territoire provient le message de demande, s'il existe déjà des considérations de risque par rapport à ce territoire, si des unités mobiles se trouvent déjà à proximité du territoire et s'il est possible d'atteindre le territoire correspondant dans un temps estimable avec l'unité mobile, c'est-à-dire un véhicule,
puis vérification de la disponibilité et du lieu où se trouvent les unités mobiles pour confier un échantillonnage ou des tests,
sélection d'une des unités mobiles et dépôt d'une commande de déplacement à l'unité sélectionnée ainsi que transfert d'une directive de route à l'unité mobile, surveillance du trajet de déplacement de l'unité mobile respective par la centrale de gestion de flotte et envoi d'informations à la personne naturelle respective ainsi qu'un message d'une heure d'arrivée prévue pour éviter des temps d'attente et des contacts inutiles avec d'autres personnes,
surveillance en parallèle d'éventuels autres messages de demande arrivés et établissement d'un plan de délais pour la gestion de flotte par adaptation de la route de déplacement afin d'optimiser l'intervention du véhicule, réduction du temps de déplacement et prise de contact rapide avec les personnes à tester,
détermination d'un lieu de collecte pour une prise en charge brève, pertinente pour les échantillons de la personne naturelle concernée dans l'unité mobile,
exécution de l'échantillonnage et/ou du test nécessaire et saisie et vérification des données relatives à la personne ainsi que transfert de ces données dans un registre central ou le cloud,
évaluation de l'échantillonnage ou du test d'ores et déjà dans l'unité mobile pendant le déplacement conformément à la directive de route,
transfert du résultat de l'échantillonnage ou du test dans le registre central ou le cloud,
adaptation de la directive de route à l'aide des directives actuelles par la centrale de gestion de flotte, au cours du temps d'intervention de l'unité mobile,
réalisation d'autres cycles d'échantillonnage et/ou de tests par démarrage selon la directive de route et fréquentation des lieux de collecte ainsi que support technique et hygiénique pour l'unité mobile afin de rétablir la capacité d'intervention pour un cycle d'intervention suivant par nettoyage et désinfection.

2. Procédé selon la revendication 1, **caractérisé par** l'utilisation d'une pluralité d'unités mobiles pour exécuter des prélèvements pour les tests sans intervention humaine par utilisation d'un robot mécanique.

3. Dispositif comprenant des moyens configurés pour exécuter un procédé selon la revendication 2, **caractérisé par** un véhicule avec montage modulaire pour recevoir des prélèvements et des tests ainsi que la réception temporaire de patients et de personnel de laboratoire, dans lequel un espace de conduite, un espace de laboratoire et un espace pour patients séparés physiquement en secteurs sont formés.

4. Dispositif selon la revendication 3, **caractérisé par** un système de réception produisant peu de vibrations pour intégrer des appareils médicaux destinés à être utilisés en exploitation mobile.

5. Dispositif selon la revendication 3 ou 4, **caractérisé en ce que** le dispositif présente des moyens pour transmettre des résultats de tests et des données de patients directement sur un terminal mobile du patient.
